(19) **Europäisches Patentamt / European Patent Office / Office européen des brevets**

(11) **EP 1 733 720 B1**

(12) **EUROPEAN PATENT SPECIFICATION**

(45) Date of publication and mention
of the grant of the patent:
**02.05.2012 Bulletin 2012/18**

(51) Int Cl.:
*A23L 1/30* (2006.01)    *A61K 31/09* (2006.01)
*A61K 31/122* (2006.01)    *A61K 47/06* (2006.01)
*A61K 47/14* (2006.01)    *A61K 47/24* (2006.01)
*A61K 47/44* (2006.01)

(21) Application number: **05728842.5**

(22) Date of filing: **04.04.2005**

(86) International application number:
**PCT/JP2005/006592**

(87) International publication number:
**WO 2005/097091 (20.10.2005 Gazette 2005/42)**

(54) **COMPOSITION COMPRISING REDUCED-FORM COENZYME Q10 AND CAROTENOID COMPOUND**

ZUSAMMENSETZUNG MIT DER REDUZIERTEN FORM VON COENZYM Q10 UND CAROTENOID-VERBINDUNG

COMPOSITION COMPRENANT LE COENZYME Q10 ET UN COMPOSE DE CAROTENOIDE

(84) Designated Contracting States:
**AT BE BG CH CY CZ DE DK EE ES FI FR GB GR HU IE IS IT LI LT LU MC NL PL PT RO SE SI SK TR**

(30) Priority: **09.04.2004 JP 2004116054**
**09.09.2004 JP 2004262975**

(43) Date of publication of application:
**20.12.2006 Bulletin 2006/51**

(73) Proprietor: **KANEKA CORPORATION**
**Osaka-shi, Osaka 530-8288 (JP)**

(72) Inventors:
• **KITAMURA, Shiro**
**Akashi-shi, Hyogo 673-0882 (JP)**

• **UEDA, Takahiro**
**Kobe-shi, Hyogo 6550872 (JP)**
• **FUJII, Kenji**
**Kobe-shi,**
**Hyogo 6511202 (JP)**
• **UEDA, Yasuyoshi**
**Himeji-shi, Hyogo 6711227 (JP)**

(74) Representative: **HOFFMANN EITLE**
**Patent- und Rechtsanwälte**
**Arabellastraße 4**
**81925 München (DE)**

(56) References cited:
**WO-A1-01/52822        WO-A1-03/061395**
**JP-A- 2003 026 625    JP-A- 2003 119 126**
**JP-A- 2003 119 127**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

The present invention relates to compositions, which contain both reduced coenzyme $Q_{10}$ and astaxanthin and have high antioxidative activities, and their use in methods for removing active oxygen and/or free radicals. Reduced coenzyme $Q_{10}$ and carotenoids are useful compounds as foods, functional nutritive foods, specified health foods, nutritious supplements, nutritional supplements, veterinary drugs, drinks, feed, cosmetics, drugs, therapeutic medicines, preventive medicines, and so forth.

2. Description of the Related Art

Coenzyme Q is an essential and ubiquitous component of living organisms, from bacteria to mammals, and is identified as a constituent of the electron transport system in mitochondria of the organism cells. Coenzyme Q functions as a transferring component in the electron transport system by repeating oxidation and reduction in mitochondria. In humans, a main component of coenzyme Q is coenzyme $Q_{10}$, which has ten repeating structural units in its side chains. Coenzyme $Q_{10}$ is present both in an oxidized form and a reduced form in vivo and functions in a predetermined ratio of the oxidized form to the reduced form. It is believed that about 40% to 90% of coenzyme $Q_{10}$ in vivo is generally of a reduced form. Coenzyme Q has physiological activities for, e.g. enhancing energy production through the activation of mitochondria, enhancing cardiac function, stabilizing cellular membranes, and protecting cells by antioxidative activity.
As described above, coenzyme $Q_{10}$ is present in the oxidized or reduced form. Reduced coenzyme $Q_{10}$ has an antioxidative activity, and administration of a sufficient amount of reduced coenzyme $Q_{10}$ into blood may effectively increase the antioxidative activity in the blood. An increase in the antioxidative activity in the blood may effectively act on many diseases that are suggested to be exacerbated by active oxygen species. Specifically, disorders such as angiopathy induced by ischemia reperfusion, restenosis in arteriosclerosis, angiopathy after cerebral infarction, arteriosclerosis, and complications of diabetes mellitus may be prevented by the increased antioxidative activity.
Carotenoids are pigments that are widely present in nature, e.g. in leaves and fruits of plants, vegetables, and algae. It is suggested that carotenoids protect living organisms from active oxygen that is generated during the photosynthesis of chlorophyll in the plant or alga cells. Carotenoids are also distributed widely in animals that ingest these plants, and are also contained in shells of Crustacea such as crab, shrimp, and crawfish, muscles of salmon, echinoid, eggs of Theragra chalcogramma, and so forth. Some report that the hatchability of eggs having a low content of carotenoid is significantly decreased. Though the reasons for it have not yet been clarified, it is believed that carotenoids also protect animals from adverse effects caused by active oxygen species and the like.
Examples of carotenoids include lycopene, which is abundantly contained in tomatoes, β-carotene, which is abundantly contained in carrots, α-carotene, astaxanthin, cryptoxanthin, zeaxanthin, lutein, canthaxanthin, fucoxanthin, and vitamin A. It is suggested that carotenoids also have very important functions in humans who ingest such foods containing these ingredients.
Specifically, there are reports that β-carotene inhibits the development of cancer (M. M. Mathews-Roth, Pure Appl. Chem., 57: 717-722, 1984); lycopene antagonizes the progression of colon cancer (T. Narisawa, et al., Jpn. J. Cancer Res., 89: 1003-1008, 1998); lutein and zeaxanthin decrease a risk of age-related retinal macular degeneration (J. M. Seddon, et al., JAMA, 272: 1413-1420, 1994); astaxanthin changes ocular dysfunction for the better (International Publication No. WO02/094253); and astaxanthin prevents and cures cataract (Japanese Unexamined Patent Application Publication No. 10-276721). Since these carotenoids belong to tetraterpenes each having isoprene units, these carotenoids have very similar activities in vivo due to their similar structures.
It is believed that many of the effects described above are caused by antioxidative activities of reduced coenzyme $Q_{10}$ and carotenoids, in particular, caused by their activities to remove active oxygen and/or free radicals generated in living organisms. Therefore, the functions of reduced coenzyme $Q_{10}$ or carotenoids described above may be very important for living organisms, thus, the uptake of them is beneficial for the living organisms.
However, reduced coenzyme $Q_{10}$ is very unstable to air oxidation. This prevents the practical use of reduced coenzyme $Q_{10}$. Hence, the inventors found a preparation containing both reduced coenzyme $Q_{10}$ with various antioxidants to stably maintain reduced coenzyme $Q_{10}$ (see Japanese Unexamined Patent Application Publication No. 2003-19127). EP 1 474 991 A1 describes an ubiquinol-enriched oil/fat-containing food which may further contain an antioxidant or an edible color such as ascorbyl palmitate, ascorbyl stearate, catechin, lecithin, tocopherol, tocotrienol, lignan or a carotenoid. However, in some cases, such stabilized reduced coenzyme $Q_{10}$ is still insufficient in antioxidative activity, namely, cannot sufficiently remove active oxygen and/or free radicals. Therefore, a method for improving the activity and a preparation having such an activity have been required.

On the other hand, contrary to expectation, carotenoids cannot exhibit adequate activities to remove active oxygen and/or free radicals generated in the living organisms. Therefore, the above-mentioned activities in vivo are not sufficiently performed.

## SUMMARY OF THE INVENTION

It is an object of the present invention to provide compositions which more effectively exhibit activities of reduced coenzyme $Q_{10}$ or carotenoids to remove active oxygen and/or free radicals, and to provide a method for removing active oxygen and/or free radicals by using the composition.

The inventors have intensively studied to solve the above-mentioned problems and have completed the present invention from remarkable finding that a combined treatment of reduced coenzyme $Q_{10}$ and a carotenoid shows a synergistic activity for removing the active oxygen and/or free radicals, compared with that when they are used separately. The present invention relates to:

(1) A composition having a synergistically enhanced activity for removing active oxygen and/or free radicals comprising reduced coenzyme $Q_{10}$ and a carotenoid, wherein the carotenoid is astaxanthin, and wherein the ratio by weight of the carotenoid to the reduced coenzyme $Q_{10}$ is 0.01 or more.

(2) The composition according to (1), wherein the ratio by weight of the carotenoid to the reduced coenzyme $Q_{10}$ ranges from 0.01 to 10.

(3) The composition according to (1), further comprising oil and/or fat.

(4) The composition according to (3), wherein the weight of reduced coenzyme $Q_{10}$ to the oil and/or fat is 0.1% or more.

(5) The composition according to any one of (1) to (4), wherein the composition is processed into an orally-administered form.

(6) The composition according to any one of (1) to (4), wherein the composition is in a form to be directly administered to skin.

(7) An orally-administered agent comprising the composition according to any one of (1) to (4) as active component.

(8) A skin agent comprising the composition according to any one (1) to (4) as an active component.

(9) A method for preventing oxidation of foods, drugs, or cosmetics by adding a composition as defined in any one of (1) to (4) into the foods, drugs, or cosmetics.

(10) A non-therapeutic method of removing active oxygen and/or free radicals by using the composition according to any one of (1) to (4).

According to the present invention, the improved composition having a high activity for removing active oxygen and/or free radicals is provided.

## DESCRIPTION OF THE PREFERRED EMBODIMENTS

The present invention will now be described in detail. A composition according to the present invention contains both reduced coenzyme $Q_{10}$ and astaxanthin as essential components. By containing both reduced coenzyme $Q_{10}$ and the carotenoid astaxanthin, the antioxidative activity of the composition, i.e. the activity for removing active oxygen and/or free radicals, is synergistically enhanced.

Here, the term "the activity for removing active oxygen and/or free radicals is synergistically enhanced" means that an antioxidative activity (A) in combination of reduced coenzyme $Q_{10}$ and a carotenoid is higher than the sum of an antioxidative activity (B) of reduced coenzyme $Q_{10}$ alone and an antioxidative activity (C) of the carotenoid alone. The activities are measured by a method described below in embodiments. The synergistic activity is evaluated by the following formula I:

$$A/(B + C) \times 100 \qquad \text{(formula I)}$$

The value higher than 100 means synergistically enhanced activity. It is preferable that a value is 105 or more and more preferably 110 or more.

The synergistic activity may be also defined by the antioxidative activity of reduced coenzyme $Q_{10}$ in combination of reduced coenzyme $Q_{10}$ and a carotenoid higher than that of reduced coenzyme $Q_{10}$ alone. Therefore, the synergistic activity is also evaluated by the following formula II:

$$(A - C)/B \times 100 \qquad \text{(formula II)}$$

The value higher than 100 means synergistically enhanced activity. It is preferable that a value is 105 or more, more preferably 110 or more, further more preferably 120 or more, and most preferably 130 or more.

Reduced coenzyme $Q_{10}$ according to the present invention can be obtained by known methods such as synthesis, fermentation, and extraction from natural products. Preferably, reduced coenzyme $Q_{10}$ can be obtained by reducing oxidized coenzyme $Q_{10}$ such as existing high-purity coenzyme $Q_{10}$ or a mixture of oxidized coenzyme $Q_{10}$ and reduced coenzyme $Q_{10}$ by a general reducing agent, e.g. sodium hydrosulfite (sodium hyposulfite), sodium borohydride, and ascorbic acid.

When the ratio of reduced coenzyme $Q_{10}$ in the composition is too low, a required antioxidative activity cannot be achieved. Therefore, the ratio of reduced coenzyme $Q_{10}$ in the composition is usually 0.01 wt% or more. Preferably, the ratio is 0.1 wt% or more, more preferably 1 wt% or more, further more preferably 10 wt% or more, even more preferably 30 wt% or more, and most preferably 60 wt% or more. Although the upper limit of the reduced coenzyme $Q_{10}$ content is not defined in the present invention, the ratio of reduced coenzyme $Q_{10}$ is usually 90 wt% or less, preferably 80 wt% or less, and more preferably 70 wt% or less, from the viewpoint of cost.

Reduced coenzyme $Q_{10}$ according to the present invention can be used alone. However, when reduced coenzyme $Q_{10}$ is used as a mixture with oxidized coenzyme $Q_{10}$, it is preferable that the ratio of reduced coenzyme $Q_{10}$ to the total of coenzyme $Q_{10}$ be 60 wt% or more, more preferably 70 wt% or more, further more preferably 80 wt% or more, even more preferably 90 wt% or more, and most preferably 95 wt% or more. The upper limit of the ratio is usually 99.9 wt% or less, although the theoretical upper limit is 100%. The astaxanthin may be chemically synthesized, or may be extracted from natural products and then purified.

Examples of the natural products containing these carotenoids include algae, yeasts, phytoplankton, fruits, vegetables, herbs, Crustacea, and eggs.

The composition according to the present invention contains both reduced coenzyme $Q_{10}$ and the carotenoid astaxanthin. The ratio by weight of the carotenoid to reduced coenzyme $Q_{10}$ is usually 0.01 or more, and preferably 0.05 or more. Although the upper limit is not defined in the present invention, it is usually 10 or less, preferably 1 or less, and more preferably 0.1 or less.

The composition according to the present invention may include oils and fats. Such oils and fats may be natural oils and fats from plants and animals, or may be synthetic or modified oils and fats. Examples of the vegetable oils and fats include coconut oil, palm oil, palm kernel oil, flaxseed oil, camellia oil, rice germ oil, rapeseed oil, rice oil, peanut oil, corn oil, wheat germ oil, soybean oil, perilla oil, cottonseed oil, sunflower seed oil, kapok oil, evening primrose oil, shea butter, sal fat, cacao butter, sesame oil, safflower oil, and olive oil. Examples of the animal oils and fats include lard, butterfat, fish oil, and tallow. Furthermore, modified oils and fats of the natural oils and fats by fractionation, hydrogenation, transesterification, or the like, for example, hydrogenated oils, are also included. Middle-chain fatty acid triglycerides (MCT), partial glycerides of fatty acids, propylene glycol fatty acid esters, phospholipids, and the like can be also used. Mixtures of these oils and fats can be used, too.

Examples of the middle-chain fatty acid triglycerides include triglycerides of fatty acids each having 6 to 12 carbon atoms, preferably 8 to 12 carbon atoms. Examples of the partial glycerides of fatty acids include monoglycerides and diglycerides of fatty acids each having 6 to 18 carbon atoms, preferably 6 to 12 carbon atoms.

Examples of propylene glycol fatty acid esters include monoglycerides and diglycerides of fatty acids each having 6 to 18 carbon atoms, preferably 6 to 12 carbon atoms.

Examples of phospholipids include egg-yolk lecithin, purified soybean lecithin, phosphatidylcholine, phosphatidylethanolamine, phosphatidylserine, sphingomyelin, dicetylphosphoric acid, stearylamine, phosphatidylglycerol, phosphatidic acid, phosphatidylinositolamine, cardiolipin, ceramide phosphorylethanolamine, ceramide phosphorylglycerol, and mixtures thereof.

Among the above-mentioned oils and fats, from the viewpoint of the ease of use and odor, vegetable oils and fats, synthetic or modified oils and fats, and phospholipids are preferable. Preferably, these oils and fats are selected in consideration of the cost and stability of reduced coenzyme $Q_{10}$ and carotenoids. For example, preferable oils and fats are coconut oil, palm oil, palm kernel oil, rapeseed oil, rice oil, soybean oil, cottonseed oil, olive oil, safflower oil, MCT, and phospholipids. Rice oil, soybean oil, rapeseed oil, safflower oil, MCT, and phospholipids are most preferable.

The ratio by weight of reduced coenzyme $Q_{10}$ to these oils and fats is usually 0.1% or more, preferably 1% or more, and more preferably 10% or more. Although the upper limit of the ratio is not defined in the present invention, it is usually 90% or less, preferably 80% or less, and more preferably 70% or less.

The composition according to the present invention may contain components other than reduced coenzyme $Q_{10}$ and carotenoids. Examples of such components include, but are not limited to, excipients, disintegrants, lubricants, binders, antioxidants, colorants, agglomeration inhibitors, absorption promoters, solubilizers for active ingredients, and stabilizers.

Examples of the excipients include, but are not limited to, sucrose, lactose, glucose, cornstarch, mannitol, crystalline cellulose, calcium phosphate, and calcium sulfate.

Examples of the disintegrants include, but are not limited to, starch, agar, calcium citrate, calcium carbonate, sodium hydrogencarbonate, dextrin, crystalline cellulose, carboxymethyl cellulose, and tragacanth.

Examples of the lubricants include, but are not limited to, talc, magnesium stearate, polyethylene glycol, silica, and hydrogenated vegetable oils.

Examples of the binders include, but are not limited to, ethyl cellulose, methylcellulose, hydroxypropyl methylcellulose, tragacanth, shellac, gelatin, gum arabic, polyvinylpyrrolidone, polyvinyl alcohol, polyacrylic acid, polymethacrylic acid, and sorbitol.

Examples of the antioxidants include, but are not limited to, ascorbic acid, tocopherol, sodium hydrogensulfite, sodium thiosulfate, sodium pyrosulfite, and citric acid.

Examples of the colorants include, but are not limited to, colorants that are approved as additives for drugs or foods.

Examples of the agglomeration inhibitors include, but are not limited to, stearic acid, talc, light anhydrous silicic acid, and hydrous silicon dioxide.

Examples of the absorption promoters include, but are not limited to, higher alcohols, higher fatty acids, and surfactants such as sucrose fatty acid ester, sorbitan fatty acid ester, polyoxyethylene sorbitan fatty acid ester, and polyglycerol fatty acid ester. Polyglycerol fatty acid ester is most preferable from the viewpoint of stability of reduced coenzyme $Q_{10}$ and carotenoids. The lower limit of hydrophile-lipophile balance (HLB) of the absorption promoters is usually 4 or more, preferably 5 or more, more preferably 6 or more, further more preferably 7 or more, and most preferably 8 or more. The upper limit of HLB is usually 12 or less, preferably 11 or less, and more preferably 10 or less.

Preferable examples of polyglycerol fatty acid ester include diglycerol monocaprate, diglycerol monolaurate, tetraglycerol monolaurate, diglycerol monooleate, diglycerol dioleate, tetraglycerol monooleate, and decaglycerol pentaoleate. Diglycerol monolaurate and diglycerol monooleate are preferable, and diglycerol monooleate is most preferable.

Examples of the solubilizers for active ingredients include, but are not limited to, organic acids such as fumaric acid, succinic acid, and malic acid.

Examples of the stabilizers include, but are not limited to, benzoic acid, sodium benzoate, and ethyl parahydroxybenzoate.

The composition according to the present invention may contain active ingredients other than reduced coenzyme $Q_{10}$ and carotenoids. Examples of these additional active ingredients include amino acids, vitamins, minerals, polyphenols, organic acids, saccharides, peptides, and proteins.

Among the above-mentioned ingredients, the absorption promoters, specifically, polyglycerol fatty acid ester can significantly enhance the in vivo absorption of reduced coenzyme $Q_{10}$ and carotenoids. Therefore, when the composition according to the present invention contains such an absorption promoter, the antioxidative activity of the composition can be efficiently enhanced.

The composition containing both reduced coenzyme $Q_{10}$ and carotenoids can be administered in an unprocessed form or in an oral-administered form such as capsules (hard capsules, soft capsules, and microcapsules), tablets, syrup, or drink. The composition may be processed into a toothpaste form. A particularly preferable form is capsules, especially, soft capsules.

Base materials of the capsules include, but are not limited to, gelatins derived from bovine bone, bovine hide, hog hide, or fishskin; food additives, e.g. carrageenan and alginic acid derived from seaweed, locust bean gum and guar gum derived from plant seeds, locust bean gum and guar gum derived from plant seeds, pullulan and curdlan derived from microorganisms; and materials for production such as celluloses.

The composition containing both reduced coenzyme $Q_{10}$ and astaxanthin according to the present invention can be added to common foods such as bread, pasta, Japanese zosui (porridge of rice and vegetable), cooked rice, cookies, crackers, cake, sweets, gum, and candy. The composition can be also added to other food products without any limitation.

The composition containing both reduced coenzyme $Q_{10}$ and astaxanthin according to the present invention can be directly administered to skin as a skin agent. Examples of the formulation include, but are not limited to, ointment, liniment, lotion, and spray (which are prepared by dissolving or dispersing the composition into an appropriate base material and mixing them into cream, paste, jerry, gel, emulsion, or liquid); cataplasm (which is prepared by dissolving or dispersing the composition into a base material and then spreading the resulting mixture on a support); and plaster and tape (which are prepared by dissolving or dispersing the composition into an adhesive agent and then spreading the resulting mixture on a support).

The composition according to the present invention and the oral or skin agents containing the composition as an active component have a synergistically enhanced activity for removing active oxygen and/or free radicals and are useful for protecting living organisms from adverse effects caused by active oxygen or free radicals.

The synergistically enhanced activity for removing active oxygen and/or free radicals by using a combination of reduced coenzyme $Q_{10}$ and astaxanthin is also effective in antioxidation of foods, drugs, and cosmetics. Namely, the foods, drugs, and cosmetics containing both reduced coenzyme $Q_{10}$ and the carotenoid can be effectively protected from oxidation.

Foods, drugs, and cosmetics that are to be protected from oxidation by the method according to the present invention are not limited. Specifically, foods, drugs, and cosmetics containing easily-oxidizable oils and fats are advantageously protected from oxidation by the composition according to the present invention.

A method for preparing foods, drugs, or cosmetics containing both reduced coenzyme $Q_{10}$ astaxanthin is not limited. Reduced coenzyme $Q_{10}$ and the carotenoid may be added during the manufacturing processes of the foods, drugs, or cosmetics, or a composition of reduced coenzyme $Q_{10}$ and the carotenoid may be added to foods, drugs, or cosmetics products preliminary manufactured.

[Examples]

The present invention will now be explained in detail with reference to examples, but the scope of the present invention is not limited to these examples.

[Measurement of antioxidative activity]

(Method 1)

In method 1 of measuring antioxidative activity, a level of thiobarbituric acid-reactive substances (TBARS) generated by an oxidative reaction was determined (Buege J. A. and Aust S.D., Methods Enzymol., 1978, 52: 302).

In this method, rat brain was homogenized in about 2.5 times by weight of saline to prepare a brain homogenate. The brain homogenate (80 $\mu$l), an ethanol solution (30 $\mu$l). containing antioxidative substance, Eagle's MEM medium (120 $\mu$l), $CuSO_4$ (240 $\mu$M), and $FeCl_3$ (240 $\mu$M) were mixed and the mixture was maintained at 37°C for 27 hours. Then, a 20% trichloroacetic acid (TCA) solution (0.9 ml) and a 0.67% thiobarbituric acid (TBA) solution (0.9 ml) were added and the resulting mixture was heated at 100°C for 30 minutes. After cooling by water, the mixture was centrifuged to separate a supernatant. The absorbance of the supernatant was measured at 532 nm. In this measuring system, the level of TBARS decreases as an antioxidative activity of an antioxidative substance increases. The antioxidative activity (%) of an antioxidative substance was determined by the ratio of a decrease of the TBARS content in the presence of an antioxidative substance to the TBARS content in the absence of an antioxidative substance.

(Method 2)

Since the TBARS is red, method 1 cannot accurately determine absorbance of some carotenoids. Therefore, spectrometry using 1,1-dipehnyl-2-picrylhydrazyl (DPPH) was also conducted (K. Shinohara and T. Suzuki, Shokuhin-Kinou-Kenkyuho (Methods for Functional Food Analysis), 2000, 218).

In method 2, 12 ml of a 200 mM MES buffer (pH 6.0) was added to 12 ml of a 400 $\mu$M DPPH ethanol solution. Then, 12 ml ethanol was added to prepare a mixture. An ethanol solution (0.6 ml) containing an antioxidative substance was added to the mixture (0.9 ml). After 20 minutes, the absorbance of the mixture was measured at 510 nm. In this measuring system, the absorbance decreases as the antioxidative activity of the antioxidative substance increases. The antioxidative activity of the antioxidative substance was quantitatively determined using a calibration curve prepared with catechin. Namely, the minimum absorbance when DPPH radicals were completely consumed with an excess amount of catechin corresponded to 100% antioxidative activity. The maximum absorbance when DPPH radicals were not consumed at all corresponded to 0% antioxidative activity. The calibration curve was drawn from these 2 points and the antioxidative activity (%) was determined from the absorbance of the antioxidative substance.

[Formula for evaluation]

According to the above-mentioned methods, the antioxidative activity (A) in combination of reduced coenzyme $Q_{10}$ and a carotenoid, the antioxidative activity (B) of reduced coenzyme $Q_{10}$ used alone, and the antioxidative activity (C) of the carotenoid used alone are measured. The antioxidative activity in combination of reduced coenzyme $Q_{10}$ and the carotenoid is compared with the sum (additive effect) of the antioxidative activity of reduced coenzyme $Q_{10}$ alone and the antioxidative activity of the carotenoid alone in order to investigate the synergistic effect.

In the present invention, both the following two formulae were used to correctly determine if the use in combination of reduced coenzyme $Q_{10}$ and a carotenoid show synergistic effect.

$$\text{Formula I: } A/(B + C) \times 100$$

$$\text{Formula II: } (A - C)/B \times 100$$

Wherein A means the antioxidative activity in combination of reduced coenzyme $Q_{10}$ and the carotenoid, B means the antioxidative activity of reduced coenzyme $Q_{10}$ used alone, and C means the antioxidative activity of the carotenoid used alone.

In formula I, an antioxidative activity in combination of reduced coenzyme $Q_{10}$ and the carotenoid is compared with the sum of the antioxidative activities when they were used alone. The theoretical value of additive effect is 100. A value above 100 means synergistic effect.

On the other hand, in formula II, the antioxidative activity of reduced coenzyme $Q_{10}$ in combination of reduced coenzyme $Q_{10}$ and the carotenoid is compared with the antioxidative activity of reduced coenzyme $Q_{10}$ used alone. The numerator in formula II is a value obtained by subtracting the antioxidative activity of carotenoid used alone from the antioxidative activity in combination of reduced coenzyme $Q_{10}$ and the carotenoid. When the use in combination merely has additive effect, the value is equal to that of reduced coenzyme $Q_{10}$ used alone. Therefore, the theoretical value in such case is 100. A value above 100 means synergistic effect.

[Reference Example 1]

Oxidized coenzyme $Q_{10}$ (100 g) and L-ascorbic acid (60 g) were added to ethanol (1,000 g) and the mixture was stirred at 78°C for a reducing reaction. After 30 hours, the mixture was cooled to 50°C. Ethanol (400 g) and water (100 g) were added to the mixture while the temperature was maintained at 50°C. The resulting ethanol solution was cooled to 2°C at a rate of 10°C/hour under stirring. The resulting wet crystals were collected by filtration and were washed with cooled ethanol and then with cooled water. The resulting wet crystals were dried under reduced pressure to yield 95 g dried white crystals (isolated product yield: 95 mol%). All procedures other than drying under reduced pressure were conducted under a nitrogen atmosphere. The ratio by weight of reduced coenzyme $Q_{10}$/oxidized coenzyme $Q_{10}$ in the resulting crystals was 99.5/0.5.

(Example 1)

Ethanol solutions containing 100 mM reduced coenzyme $Q_{10}$ prepared in Reference Example 1 and/or 100 mM astaxanthin, which is a carotenoid, were prepared. The antioxidative activity of each ethanol solution was measured by the above-mentioned method 1. Each activity was applied to formulae I and II to evaluate the synergistic effect. As shown in Table 1, the value by formula I was 135 and that by formula II was 205. Thus, the obvious synergistic effect by using both reduced coenzyme $Q_{10}$ and astaxanthin was confirmed.

Table 1

| | Antioxidative Activity (%) (Inhibition Ratio of Oxidation) | Formula I | Formula II |
| --- | --- | --- | --- |
| | | A/(B+C)×100 | (A-C)/B×100 |
| Reduced coenzyme $Q_{10}$ | 10.9 | - | - |
| Astaxanthin | 21.6 | - | - |

(continued)

| | Antioxidative Activity (%) (Inhibition Ratio of Oxidation) | Formula I | Formula II |
| --- | --- | --- | --- |
| | | A/(B+C)×100 | (A-C)/B×100 |
| Reduced coenzyme Q10 and astaxanthin | 43.9 | 135 | 205 |

A: in combination of reduced coenzyme $Q_{10}$ and astaxanthin
B: reduced coenzyme $Q_{10}$ alone
C: astaxanthin alone

(Reference Example 2)

Ethanol solutions containing 25 μM reduced coenzyme $Q_{10}$ prepared in Reference Example 1 and/or 25 μM β-carotene, and ethanol solutions containing 25 μM reduced coenzyme $Q_{10}$ and/or 25 μM lycopene were prepared. The antioxidative activity of each ethanol solution was measured by the above-mentioned method 2. Each activity was applied to formulae I and II to evaluate the synergistic effect. As shown in Table 2, in combination of reduced coenzyme $Q_{10}$ and β-carotene, the value by formula I was 107 and that by formula II was 114. In combination of reduced coenzyme $Q_{10}$ and lycopene, the value by formula I was 105 and that by formula II was 105. Thus, the obvious synergistic effect by using both reduced coenzyme $Q_{10}$ and β-carotene or by using reduced coenzyme $Q_{10}$ and lycopene was confirmed.

Table 2

| | Antioxidative Activity (%) (Inhibition Ratio of Oxidation) | Formula I | Formula II |
| --- | --- | --- | --- |
| | | $A/(B+C) \times 100$ | $(A-C)/B \times 100$ |
| Reduced coenzyme $Q_{10}$ | 10.3 | - | - |
| β-Carotene | 8.8 | - | - |
| Reduced coenzyme $Q_{10}$ and β-carotene | 20.5 | 107 | 114 |
| Lycopene | 0.9 | - | - |
| Reduced coenzyme Q10 and lycopene | 11.7 | 105 | 105 |

A: in combination of reduced coenzyme $Q_{10}$ and β-carotene or combination of reduced coenzyme $Q_{10}$ and lycopene
B: reduced coenzyme $Q_{10}$ alone
C: β-carotene or lycopene alone

(Comparative Example 1)

The same procedure as Example 1 was performed by using reduced coenzyme $Q_{10}$ prepared in Reference Example 1 and vitamin E instead of astaxanthin. The values by formulae I and II were 90 and 82, respectively. Thus, in combination of reduced coenzyme $Q_{10}$ and vitamin E, synergistic effect was not confirmed.

(Example 3)

Reduced coenzyme $Q_{10}$ prepared in Reference Example 1 and astaxanthin were added to a mixture of rice oil, hydrogenated oil, beeswax, lecithin, and diglycerol monooleate. Gelatin soft capsules containing the following components were prepared by a common method.

Reduced coenzyme $Q_{10}$: 60 parts by weight,
Astaxanthin: 10 parts by weight,
Rice oil: 500 parts by weight,
Diglycerol monooleate: 180 parts by weight,
Hydrogenated oil: 170 parts by weight,
Beeswax: 60 parts by weight, and
Lecithin: 20 parts by weight.

(Reference Example 4)

Reduced coenzyme $Q_{10}$ prepared in Reference Example 1 and vitamin A were added to middle-chain fatty acid triglyceride (MCT, $C_8$ : $C_{10}$ = 6 : 4). Gelatin soft capsules containing the following components were prepared by a common method.

Reduced coenzyme $Q_{10}$: 60 parts by weight,
Vitamin A: 5 parts by weight, and
Middle-chain fatty acid triglyceride: 935 parts by weight.

(Reference Example 5)

Reduced coenzyme $Q_{10}$ prepared in Reference Example 1 and lycopene (tomato extract) were dissolved in propanol. The resulting solution was adsorbed to microcrystalline cellulose and then was dried under reduced pressure. The dried mixture was mixed with cornstarch, lactose, carboxymethyl cellulose, and magnesium stearate under a nitrogen atmosphere. Then, the mixture was added to a polyvinylpyrrolidone aqueous solution of a binder and granulated by a common method. The resulting granules were mixed with talc of a lubricant and then were made into tablets.

Reduced coenzyme $Q_{10}$: 150 parts by weight,
Lycopene: 10 parts by weight,
Cornstarch: 200 parts by weight,
Lactose: 120 parts by weight,
Carboxymethyl cellulose: 80 parts by weight,
Microcrystalline cellulose: 300 parts by weight,
Polyvinylpyrrolidone: 40 parts by weight,
Magnesium stearate: 20 parts by weight, and
Talc: 80 parts by weight

(Example 6)

Cream of the following composition was prepared by a common method. Reduced coenzyme $Q_{10}$ prepared in Reference Example 1 was used.

Reduced coenzyme $Q_{10}$: 10 parts by weight,
Astaxanthin: 1 part by weight,
Glycerol sorbitan fatty acid ester: 60 parts by weight,
Microcrystalline wax: 10 parts by weight,
Olive oil: 30 parts by weight,
Liquid paraffin: 180 parts by weight,
Magnesium stearate: 10 parts by weight,
Propylene glycol: 37 parts by weight,
Magnesium sulfate: 7 parts by weight, and
Desalted water: 655 parts by weight.

**Claims**

1. A composition having a synergistically enhanced activity for removing active oxygen and/or free radicals, comprising reduced coenzyme $Q_{10}$ and a carotenoid, wherein the carotenoid is astaxanthin, and wherein the ratio by weight of the carotenoid to the reduced coenzyme $Q_{10}$ is 0.01 or more.

2. The composition according to claim 1, wherein the ratio by weight of the carotenoid to the reduced coenzyme $Q_{10}$ ranges from 0.01 to 10.

3. The composition according to claim 1, further comprising oil and/or fat.

4. The composition according to claim 3, wherein the weight of reduced coenzyme $Q_{10}$ to the oil and/or fat is 0.1% or more.

5. The composition according to any one of claims 1 to 4, wherein the composition is processed into an orally-administered form.

6. The composition according to any one of claims 1 to 4, wherein the composition is in a form to be directly administered to skin.

7. An orally-administered agent comprising the composition according to any one of claims 1 to 4 as active component.

8. A skin agent comprising the composition according to any one of claims 1 to 4 as an active component.

9. A method for preventing oxidation of foods, drugs, or cosmetics by adding a composition as defined in any one of claims 1 to 4 into the foods, drugs, or cosmetics.

10. A non-therapeutic method of removing active oxygen and/or free radicals by using the composition according to any one of claims 1 to 4.


**Patentansprüche**

1. Zusammensetzung mit einer synergistisch erhöhten Aktivität zum Abbau von aktivem Sauerstoff und/oder freien Radikalen, umfassend reduziertes Coenzym $Q_{10}$ und ein Carotenoid, wobei das Carotenoid Astaxanthin ist und wobei das Gewichtsverhältnis des Carotenoids zu dem reduzierten Coenzym $Q_{10}$ 0,01 oder mehr beträgt.

2. Zusammensetzung gemäß Anspruch 1, wobei das Gewichtsverhältnis des Carotenoids zu dem reduzierten Coenzym $Q_{10}$ von 0,01 bis 10 reicht.

3. Zusammensetzung gemäß Anspruch 1, die ferner Öl und/oder Fett umfasst.

4. Zusammensetzung gemäß Anspruch 3, wobei das Gewicht des reduzierten Coenzyms $Q_{10}$ zu dem Öl und/oder Fett 0,1 % oder mehr beträgt.

5. Zusammensetzung gemäß mindestens einem der Ansprüche 1 bis 4, wobei die Zusammensetzung zu einer oral verabreichenden Form verarbeitet ist.

6. Zusammensetzung gemäß mindestens einem der Ansprüche 1 bis 4, wobei die Zusammensetzung in einer direkt der Haut zu verabreichenden Form vorliegt.

7. Oral zu verabreichendes Mittel, umfassend die Zusammensetzung gemäß mindestens einem der Ansprüche 1 bis 4 als Wirkkomponente.

8. Hautmittel, umfassend die Zusammensetzung gemäß mindestens einem der Ansprüche 1 bis 4 als Wirkkomponente.

9. Verfahren zur Verhinderung der Oxidation von Lebensmitteln, Arzneimitteln oder Kosmetika durch Zugabe einer wie in einem der Ansprüche 1 bis 4 definierten Zusammensetzung zu den Lebensmitteln, Arzneimitteln oder Kosmetika.

10. Nicht-therapeutisches Verfahren zum Abbau von aktivem Sauerstoff und/oder freien Radikalen unter Verwendung der Zusammensetzung gemäß mindestens einem der Ansprüche 1 bis 4.


**Revendications**

1. Composition ayant une activité synergiquement augmentée pour éliminer l'oxygène actif et/ou les radicaux libres, comprenant un coenzyme $Q_{10}$ réduit et un caroténoïde, dans laquelle le caroténoïde est l'astaxanthine, et dans laquelle le rapport pondéral du caroténoïde au coenzyme $Q_{10}$ réduit est de 0,01 ou plus.

2. Composition selon la revendication 1, dans laquelle le rapport pondéral du caroténoïde au coenzyme $Q_{10}$ réduit va de 0,01 à 10.

**3.** Composition selon la revendication 1, comprenant en outre une huile et/ou une graisse.

**4.** Composition selon la revendication 3, dans laquelle le poids du coenzyme $Q_{10}$ réduit à l'huile et/ou la graisse est de 0,1% ou plus.

**5.** Composition selon l'une quelconque des revendications 1 à 4, dans laquelle la composition est transformée en une forme administrée par voie orale.

**6.** Composition selon l'une quelconque des revendications 1 à 4, dans laquelle la composition est sous une forme pour être directement administrée à la peau.

**7.** Agent administré par voie orale comprenant la composition selon l'une quelconque des revendications 1 à 4 comme composant actif.

**8.** Agent cutané comprenant la composition selon l'une quelconque des revendications 1 à 4 comme composant actif.

**9.** Procédé destiné à empêcher l'oxydation des aliments, des médicaments ou des cosmétiques par l'adjonction d'une composition telle que définie selon l'une quelconque des revendications 1 à 4 dans les aliments, les médicaments ou les cosmétiques.

**10.** Procédé non thérapeutique d'élimination de l'oxygène actif et/ou des radicaux libres par l'utilisation de la composition selon l'une quelconque des revendications 1 à 4.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02094253 A
- JP 10276721 A
- JP 2003019127 A
- EP 1474991 A1

**Non-patent literature cited in the description**

- **M. M. Mathews-Roth.** *Pure Appl. Chem.,* 1984, vol. 57, 717-722
- **T. Narisawa et al.** *Jpn. J. Cancer Res.,* 1998, vol. 89, 1003-1008
- **J. M. Seddon et al.** *JAMA,* 1994, vol. 272, 1413-1420
- **Buege J. A. ; Aust S.D.** *Methods Enzymol.,* 1978, vol. 52, 302